# EUROPÄISCHE PATENTSCHRIFT

(11) **EP 0 621 064 B1**
(45) Veröffentlichungstag und Bekanntmachung des Hinweises auf die Patenterteilung: **09.04.1997**
(21) Anmeldenummer: 94105874.5
(22) Anmeldetag: 15.04.1994
(51) Int. Cl.: B01D 9/00

(54) **Verfahren zum Trennen und Reinigen von Stoffen durch Schmelzkristallisation**
Device for separating and purifying materials by melt crystallization
Procédé pour la séparation et la purification de matériaux par cristallisation de fusion sous haute pression

(30) Priorität: 22.04.1993 DE 4313121
(43) Veröffentlichungstag der Anmeldung: 26.10.1994
(73) Patentinhaber: HOECHST AKTIENGESELLSCHAFT, 65929 Frankfurt am Main (DE)
(72) Erfinder: Steiner, Rudolf, Prof. Dr., D-91056 Erlangen (DE); König, Axel, Dr., D-70374 Stuttgart (DE); Rittner, Siegbert, Dr., D-64546 Mörfelden (DE)

(56) Entgegenhaltungen:
- DE-A- 3 037 045
- PATENT ABSTRACTS OF JAPAN vol. 13, no. 336 (C-623) (3684) 27. Juli 1989 & JP-A-01 115 404 (KOBE STEEL)

## Beschreibung

Die Erfindung betrifft ein Verfahren zum Trennen und Reinigen von Stoffen durch Schmelzkristallisation unter Druck.

Unter Schmelzkristallisation versteht man das Trennen und/oder Reinigen von Stoffen, bei denen aus einer Schmelze eine oder mehrere Komponenten auskristallisieren und abgetrennt werden, während andere Bestandteile im nicht erstarrten Teil der Schmelze, der sogenannten Restschmelze verbleiben.

Schmelzkristallisation ist bekannt. Die Schmelze wird bei Normaldruck gekühlt und kristallisiert aus. Vom Kristallisat wird die Restschmelze abgetrennt. Danach wird das Kristallisat wieder erhitzt bis es schwitzt und das Schwitzöl (Tropföl) abtrennt.

Nach einem anderen Verfahren (M. Moritoki, K. Kitgawa, K. Onoe und K. Kaneko in Industrial Crystallization 84, S.J. Jancic and E.J. de Jong, Elsevier Science Publisher B.V., Amsterdam, 1984) wird die Schmelze mit einem Preßstempel unter Druck gesetzt und anschließend unter Beibehaltung des Drucks durch Absenken der Temperatur kristallisiert. Nach entfernen der Restschmelze wird der Schwitzvorgang durch Absenken des Drucks eingeleitet. Nachteilig bei diesem Verfahren sind kleine, schlecht ausgebildete Kristalle, die die Feststoff-Flüssigkeits-Trennung erschweren. Hier will die Erfindung Abhilfe schaffen.

Die Aufgabe wird durch ein Verfahren der eingangs genannten Art gelöst, bei dem man
a) ein Inertgas in die Schmelze einbringt,
b) die Schmelza unter Inertgasatmosphäre unter Druck setzt, sie anschließend abkühlt, wobei sie bei Drucken von 50 bis 5000 bar kristallisiert,
c) die Restschmelze vom entstandenen Kristallisat abtrennt,
d) danach das Kristallisat durch Druckabsenkung entgast, zum Schwitzen bringt und
e) das Schwitzöl abtrennt.

Der Gasdruck kann schrittweise bis zum Enddruck erhöht und nach Abtrennen der Restschmelze stufenweise abgesenkt werden. Als Inertgas kommen insbesondere solche in Betracht, die in der jeweiligen Schmelze löslich sind. Für die Stoffsysteme Naphthalin/Biphenvl und p-/m-Dichlorbenzol eignen sich z. B. Stickstoff, Kohlendioxid, Ethan, Ethen, Propan etc. oder deren Gemische. Zwecks besserer Löslichkeit kann das Gas unter Bewegen der Schmelze über Düsen und der gleichen in die Schmelze verteilt werden. Die Schmelze kann bei Drücken von 50 bis 5000 bar bei entsprechender Temperaturabsenkung kristallisiert werden. Der erforderliche Druck hängt vom Stoffsystem und der Zusammensetzung der Komponenten ab.

Die Vorteile des Verfahrens sind im wesentlichen im Entgasen des Kristallisats zu sehen, durch das das Kristallisat aufgelockert wird, was den Schwitzprozeß wirkungsvoll verbessert. Die stufenweise Druckentspannung erlaubt Schwitzöle mit unterschiedlichem Verunreinigungsgrad abzutrennen.

Im folgenden wird die Erfindung anhand von Beispielen näher erläutert:

### Beispiel 1

In einem Autoklaven von ca. 1,5 l Inhalt, der mit 2 gegenüberliegenden schlitzförmigen Sichtfenstern versehen und für einen maximalen Betriebsdruck von 300 bar und einer Betriebstemperatur von 250°C ausgelegt ist, wurde ein Reagenzglas freischwebend aufgehängt. In das Reagenzglas wurden etwa 10 g eines Gemisches aus 66 Gew.-% Naphthalin und 34 Gew.-% Biphenyl eingefüllt, das in einer Stickstoffatmosphäre bei einem Druck von 150 bar bei Temperaturen oberhalb 61°C schmelzflüssig vorlag. Die Temperatur wurde mit einem Thermoelement in der Nähe der zu untersuchenden Probe gemessen. Die Temperaturregelung erfolgte über die Heizung des Autoklaven mittels eines Flüssigkeitsthermostaten. Beim Abkühlen zeigten sich erste Kristalle bei 60,4°C, bei 59,8°C war etwa 1/6, bei 58,3°C etwa 9/10 des Volumens mit Kristallen durchsetzt. Bei 54°C war die gesamte Schmelze "fest'', d. h. es war keine Restschmelze zu erkennen. Der Druck wurde dann stufenweise herabgesetzt. Bei ca. 100 bar zeigten sich die ersten Gasblasen, bei 65 bar wurden die Kristalle trüb, bei 30 bar bildete sich eine flüssige Phase, wobei die Temperatur inzwischen auf 49,5°C gefallen war. Bei 1 bar und 38,7°C erfüllte die flüssige Schicht schließlich etwa 1/5 des Reagenzglasvolumens. Die Restschmelze begann bei weiterer Abkühlung erst unterhalb 38°C langsam zu kristallisieren. Die Analyse zeigte deutliche Unterschiede in der Zusammensetzung der beiden Phasen: das Kristallisat enthielt etwa 75 Gew.-%, die separierte Restschmelze etwa 46 Gew.-% Naphthalin.

Ein bei Normaldruck ausgeführter Referenzversuch ergab, daß die Kristallisation der Ausgangsschmelze bei 57,0°C beginnt und nach Abkühlen der Temperatur unterhalb 50°C keine Abtrennung der Restschmelze mehr möglich ist.

### Beispiel 2

Ein Autoklav von 0,2 l Inhalt mit einer innen angeordneten Wärmetauscherplatte wurde so auf einem Gestell montiert, daß er während des Versuchs um die Gestellachse gedreht werden konnte. Er besitzt einen elektrisch beheizbaren Heizmantel und die Wärmetauscherplatte ist mit einem Flüssigkeitsthermostat versehen. In Kristallisierstellung befindet sich die Wärmetauscherplatte im unteren Teil und ist von Schmelze umgeben. Nach Beendigung der Kristallisation wird der Autoklav gekippt, so daß die Platte in den oberen Teil gelangt damit die Restschmelze bzw. die Schwitzölfraktion ablaufen kann. In den Autoklaven wurden 50 g eines Gemisches mit 70 Gew.-% Naphthalin und 30 Gew.-% Biphenyl eingefüllt und der Wärmetauscher in das sich im unteren Teil des Autoklaven befindende Gemisch eingetaucht. Der Druck wurde im Autoklaven unter Stickstoffatmosphäre auf 100 bar eingestellt und der Autoklaveninhalt auf 75°C aufgeheizt. Die Wärmetauscherplatte wurde auf die gleiche Temperatur eingeregelt. Unter diesen Bedingungen wurde die Schmelze 1 Stunde lang temperiert und unter Bewegen des Autoklaven mit Gas gesättigt. Dann wurde die Temperatur des Wärmetauschers auf 50°C und 1 Stunde später auch die Autoklaventemperatur auf diesen Wert gesenkt. Nach einer weiteren Stunde wurde die Apparatur so gedreht, das sich die Wärmetauscherplatte nun oben befand und die Restschmelze ablaufen konnte. Dann wurde der Druck in Intervallen von 10 bar pro 5 Minuten bis auf Normaldruck abgesenkt, um eingeschlossene Anteile der Restschmelze und Schwitzöl aus dem Kristallverband herauszuholen. Anschließend wurde der Autoklav auf Raumtemperatur abgekühlt.

Die Wärmetauscherplatte war von einem Kristallisat umgeben, dessen mittlere Zusammensetzung etwa 83 Gew.-% Naphthalin enthielt. Der Naphthalin-Gehalt im Inneren der Kristallisatschicht war mit bis zu 89 Gew.-% deutlich höher als in der Außenschicht mit etwa 73 Gew.-%. Am Boden des Autoklaven hatte sich eine Schicht Restschmelze angesammelt, die einen Anteil von etwa 50 Gew.-% Naphthalin aufwies.

Bei einem Referenzversuch unter Normaldruck sammelte sich am Boden des Druckgefäßes hingegen keine Restschmelze an.

### Beispiel 3

In den Autoklaven mit Wärmetauscherplatte gemäß Beispiel 2 wurden 68 g eines Gemisches aus 88 Gew.-% p-Dichlorbenzol und 12 Gew.-% m-Dichlorbenzol eingefüllt. Dieses Gemisch wurde bei 65°C unter Stickstoffatmosphäre unter einem Druck von 100 bar gesetzt. Die Kristallisation begann bei 48°C, sie endete bei 30°C. Nach der anschließenden Druckentspannung wurden ca. 48 g Kristallisat mit einer Reinheit von 99,95 Gew.-% p-Dichlorbenzol erhalten, während die Restschmelze etwa 62 Gew.-% p-Dichlorbenzol enthielt.

Aus zwei Referenzversuchen bei Normaldruck resultierten Reinheiten des Kristallisats von 99,41 Gew.-% bzw. 99,63 Gew.-%. Die Reinheiten des Kristallisats nach der verfahrensweise mit Druckentspannung lagen nahezu eine 10er Potenz höher als die Reinheiten nach den Referenzversuchen unter Normaldruck.

### Beispiel 4

Bei einem weiteren Versuch, diesmal mit einem anorganischen Stoff, der ebenfalls im Autoklaven mit der Wärmetauscherplatte durchgeführt wurde, sind 60 g eines Gemisches eingesetzt worden, welches aus 94 % phosphoriger Säure und 6 % verschiedener geradkettiger Fettsäuren mit einer Kohlenstoffkettenlänge von C₁₂ bis C₁₆ bestand. Dieses Gemisch wurde unter einem Stickstoffstrom von 50 bar kristallisiert. Die Ausgangstemperatur lag bei 69°C (Schmelze), der Kristallisationsbeginn bei 60°C, das Kristallisationsende bei 40°C. Nach der anschließenden Druckentspannung wurden 44 g Kristallisat mit einer Reinheit von 99,6 % phosphoriger Säure gewonnen. Ein Referenzversuch bei Normaldruck erbrachte zwar auch eine Trennung in Kristallisat und Restschmelze, jedoch lag die Reinheit des Kristallisats mit 97,7 % wesentlich niedriger als beim Druckversuch mit anschließender Endgasung.

## Patentansprüche

1. Verfahren zum Trennen und Reinigen von Stoffen durch Schmelzkristallisation unter Druck, dadurch gekennzeichnet, daß
a) man ein Intergas in die Schmelze bringt,
b) die Schmelze unter Inertgasatmosphäre unter Druck setzt, sie anschließend abkühlt, wobei sie beim Drücken von 50 bis 5000 bar kristallisiert,
c) die Restschmelze vom entstandenen Kristallisat abtrennt,
d) danach das Kristallisat durch Absenken des Drucks entgast, zum Schwitzen bringt und
e) das Schwitzöl (Tropföl) abtrennt.

2. Verfahren nach Anspruch 1, dadurch gekennzeichnet, daß die Schmelze unter Stickstoff-, Kohlendioxid-, Ethan-, Ethen-, Propan-Atmosphäre oder einer Atmosphäre aus deren Gemische unter Druck gesetzt wird.

3. Verfahren nach Anspruch 1, dadurch gekennzeichnet, daß das Inertgas unter gleichzeitigem Bewegen der Schmelze in diese eingedüst wird.

## Claims

1. A process for separating and purifying substances by crystallization from the melt under pressure, wherein
a) an inert gas is introduced into the melt,
b) the melt is subjected to pressure under an inert gas atmosphere and is then cooled, whereupon it crystallizes at pressures of 50 to 5,000 bar,
c) the residual melt is separated from the resulting crystals,
d) the crystals are then degassed by lowering the pressure and are caused to sweat and
e) the sweat oil (drip oil) is separated off.

2. The process as claimed in claim 1, wherein the melt is subjected to pressure under a nitrogen, carbon dioxide, ethane, ethene or propane atmosphere or an atmosphere comprising a mixture thereof.

3. The process as claimed in claim 1, wherein the inert gas is introduced into the melt via nozzles while simultaneously moving the melt.

## Revendications

1. Procédé de séparation et de purification de matières par cristallisation par fusion sous pression, caractérisé en ce que
a) on introduit un gaz inerte dans la matière fondue,
b) on applique une pression à la matière fondue, sous une atmosphère de gaz inerte, ensuite on la refroidit, pendant qu'elle cristallise à une pression de 50 à 5 000 bars,
c) on sépare la matière fondue résiduelle de la matière cristallisée formée,
d) ensuite on dégaze la matière cristallisée par réduction de la pression, on la porte au ressuage, et
e) on sépare l'huile de ressuage.

2. Procédé selon la revendication 1, caractérisé en ce que l'on applique une pression à la matière fondue sous une atmosphère d'azote, de dioxyde de carbone, d'éthane, d'éthène, de propane ou de leurs mélanges.

3. Procédé selon la revendication 1, caractérisé en ce que le gaz inerte est introduit à l'aide d'une tuyère, tout en remuant la matière fondue.
